(19) Europäisches Patentamt
European Patent Office
Office européen des brevets

(11) EP 1 028 112 A1

(12) **EUROPÄISCHE PATENTANMELDUNG**

(43) Veröffentlichungstag:
**16.08.2000 Patentblatt 2000/33**

(21) Anmeldenummer: **00109077.8**

(22) Anmeldetag: **07.04.1997**

(51) Int. Cl.$^7$: **C07D 213/74**, C07D 213/70, C07D 213/75, C07C 233/22, C07D 233/84, C07D 215/36, C07D 235/28, C07D 239/95, A61K 31/44, A61K 31/41, A61K 31/47, A61K 31/165

(84) Benannte Vertragsstaaten:
**AT BE CH DE DK ES FI FR GB GR IE IT LI LU MC NL PT SE**

(30) Priorität: **18.04.1996 DE 19615262**

(62) Dokumentnummer(n) der früheren Anmeldung(en) nach Art. 76 EPÜ:
**97105705.4 / 0 802 188**

(71) Anmelder:
**Bayer Aktiengesellschaft**
**51368 Leverkusen (DE)**

(72) Erfinder:
- **Goldmann, Siegfried, Dr.**
  **42327 Wuppertal (DE)**
- **Müller, Ulrich, Dr.**
  **42111 Wuppertal (DE)**
- **Connell, Richard, Dr.**
  **Trumbull, CT 06611 (US)**
- **Bischoff, Hilmar, Dr.**
  **42113 Wuppertal (DE)**
- **Denzer, Dirk, Dr.**
  **42719 Solingen (DE)**
- **Grützmann, Rudi, Dr.**
  **42657 Solingen (DE)**
- **Beuck, Martin, Dr.**
  **40699 Erkrath (DE)**

Bemerkungen:
Diese Anmeldung ist am 02 - 05 - 2000 als Teilanmeldung zu der unter INID-Kode 62 erwähnten Anmeldung eingereicht worden.

(54) **Heteroverknüpfte Phenylglycinolamide als antitherosklerotische Arzneimittel**

(57) Die vorliegende Erfindung betrifft heteroverknüpfte Phenylglycinolamide, Verfahren zu ihrer Herstellung und ihre Verwendung als Arzneimittel, insbesondere als anti-atherosklerotische Arzneimittel.

EP 1 028 112 A1

## Beschreibung

Die vorliegende Erfindung betrifft heteroverknüpfte Phenylglycinolamide, Verfahren zu ihrer Herstellung und ihre Verwendung als Arzneimittel, insbesondere als anti-atherosklerotische Arzneimittel.

Es ist bekannt, dass erhöhte Blutspiegel von Triglyzeriden (Hypertriglyzeridämie) und Cholesterin (Hypercholesterinämie) mit der Genese von atherosklerotischen Gefäßwand-Veränderungen und koronaren Herzkrankheiten assoziiert sind.

EP 344 519 offenbart substituierte 4-(Chinolin-2-yl-methoxy)-phenylessigsäure-Derivate, die als Wirkstoffe in Arzneimitteln eingesetzt werden können. Die Stoffe wirken besonders als Hemmer von enzymatischen Reaktionen im Rahmen des Arachidon-Stoffwechsels, insbesondere der 5-Lipoxygenase.

Ein deutlich erhöhtes Risiko für die Entwicklung koronarer Herzerkrankungen liegt darüber hinaus vor, wenn diese beiden Risikofaktoren kombiniert auftreten, was wiederum mit einer Überproduktion an Apoliprotein B-100 einhergeht. Es besteht daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft heteroverknüpfte Phenylglycinolamide der allgemeinen Formel (I)

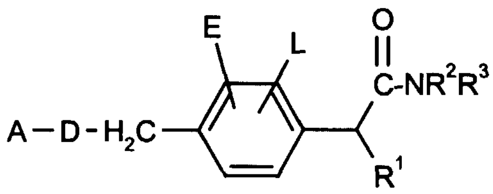

(I),

in welcher

A für Aryl mit 6 bis 10 Kohlenstoffatomen, Benzyl oder für einen gegebenenfalls benzokondensierten 5- bis 7-gliedrigen gesättigten partiell ungesättigten oder ungesättigten Heterocyclus mit bis zu 3 Heteroatomen aus der Reihe S, N und/oder O steht, wobei die Ringsysteme gegebenenfalls - auch über die N-Funktion - bis zu 3-fach gleich oder verschieden durch Halogen, Trifluormethyl, Carboxy, Hydroxyl, Nitro, Cycloalkyl mit 3 bis 7 Kohlenstoffatomen, Benzyl, Phenyl, Benzyloxy oder durch geradkettiges oder verzweigtes Alkyl, Alkoxy oder Alkoxycarbonyl mit jeweils bis zu 6 Kohlenstoffatomen substituiert sind, oder

für einen Rest der Formel $R^5R^4N$- oder

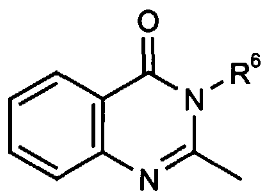

steht,

worin
$R^4$ und $R^5$ gleich oder verschieden sind und Wasserstoff, Phenyl oder geradkettiges oder verzweigtes Alkyl mit bis zu 6 Kohlenstoffatomen bedeuten,
$R^6$ Wasserstoff, Phenyl oder geradkettiges oder verzweigtes Alkyl mit bis zu 4 Kohlenstoffatomen bedeutet,

D für ein Sauerstoffatom oder für einen Rest der Formel -CO-, $-(CO)_a-NR^7$, $-(CH_2)_bS-$, $-(CH_2)_c-NR^8$ oder -CH=CH- steht,
worin

a, b und c gleich oder verschieden sind und eine Zahl 0 oder 1 bedeuten,
$R^7$ und $R^8$ gleich oder verschieden sind und Wasserstoff, geradkettiges oder verzweigtes Alkyl oder

Acyl mit jeweils bis zu 6 Kohlenstoffatomen, Phenyl oder Benzyl bedeuten, wobei die Ringsysteme gegebenenfalls bis zu 2-fach gleich oder verschieden durch Nitro, Halogen, Trifluormethyl oder durch geradkettiges oder verzweigtes Alkyl oder Alkoxy mit jeweils bis zu 3 Kohlenstoffatomen substituiert sind,

E und L gleich oder verschieden sind und für Wasserstoff, Cycloalkyl mit 3 bis 8 Kohlenstoffatomen, Azido, Hydroxy, Halogen, geradkettiges oder verzweigtes Alkyl, Alkoxy oder Alkenyl mit jeweils bis zu 6 Kohlenstoffatomen stehen,

$R^1$ für Cycloalkyl mit 3 bis 8 Kohlenstoffatomen steht, oder für geradketiges oder verzweigtes Alkyl mit bis zu 10 Kohlenstoffatomen steht,

$R^2$ für Wasserstoff oder für geradkettiges oder verzweigtes Alkyl mit bis zu 4 Kohlenstoffatomen steht,

$R^3$ für einen Rest der Formel

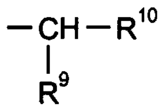

steht,

worin

$R^9$ Wasserstoff oder einen Rest der Formel $CH_2$-OH bedeutet,

$R^{10}$ Phenyl bedeutet, das gegebenenfalls bis zu 3-fach gleich oder verschieden durch Hydroxy, Halogen oder geradkettiges oder verzweigtes Alkyl mit bis zu 5 Kohlenstoffatomen substituiert ist,

und deren Salze.

Die erfindungsgemäßen heteroverknüpften Phenylglycinolamide können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren als auch Diastereomeren oder deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten, partiell ungesättigten oder ungesättigten 5-bis 7-gliedrigen, vorzugsweise 5- bis 6-gliedrigen Heterocyclus der bis zu 3 Heteroatome aus der Reihe S, N und/oder O enthalten kann und der im Fall eines Stickstoffatoms auch über dieses gebunden sein kann. Beispielsweise seien genannt: Indolyl, Chinolyl, Benzo[b]thienyl, Benzo[b]furyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Bevorzugt sind die erfindungsgemäßen Verbindungen der allgemeinen Formel (I), in welcher

A für Naphthyl, Phenyl, Benzyl, Pyridyl, Imidazolyl, Benzimidazolyl oder Chinolyl steht, die gegebenenfalls - auch über die N-Funktion - bis zu 3-fach gleich oder verschieden durch Fluor, Chlor, Brom, Trifluormethyl, Carboxy, Hydroxyl, Nitro, Cyclopropyl, Cylcopentyl, Cyclohexyl, Benzyl, Phenyl, Benzyloxy oder durch

geradkettiges oder verzweigtes Alkyl, Alkoxy oder Alkoxycarbonyl mit jeweils bis zu 4 Kohlenstoffatomen substituiert sind, oder für einen Rest der Formel $R^5R^4N$- oder

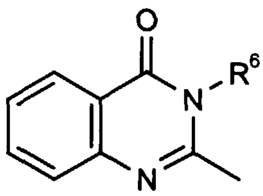

steht,

worin

$R^4$ und $R^5$ gleich oder verschieden sind und Wasserstoff, Phenyl oder geradkettiges oder verzweigtes Alkyl mit bis zu 4 Kohlenstoffatomen bedeuten,
$R^6$ Wasserstoff, Phenyl oder geradkettiges oder verzweigtes Alkyl mit bis zu 3 Kohlenstoffatomen bedeutet,

D für ein Sauerstoffatom oder für einen Rest der Formel -CO-, $-(CO)_a-NR^7$, $-(CH_2)_bS-$, $-(CH_2)_c-NR^8$ oder -CH=CH- steht,
worin

a, b und c gleich oder verschieden sind und eine Zahl 0 oder 1 bedeuten,
$R^7$ und $R^8$ gleich oder verschieden sind und Wasserstoff, geradkettiges oder verzweigtes Alkyl oder Acyl mit jeweils bis zu 5 Kohlenstoffatomen, Phenyl oder Benzyl bedeuten, wobei die Ringsysteme gegebenenfalls bis zu 2-fach gleich oder verschieden durch Nitro, Fluor, Chlor, Brom, Trifluormethyl oder durch geradkettiges oder verzweigtes Alkyl oder Alkoxy mit jeweils bis zu 3 Kohlenstoffatomen substituiert sind,

E und L gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 3 Kohlenstoffatomen stehen,
$R^1$ für Cyclopropyl, Cylcobutyl, Cyclopentyl, Cyclohexyl oder Cycloheptyl steht, oder für geradkettiges oder verzweigtes Alkyl mit bis zu 8 Kohlenstoffatomen steht,
$R^2$ für Wasserstoff oder für geradkettiges oder verzweigtes Alkyl mit bis zu 3 Kohlenstoffatomen steht,
$R^3$ für einen Rest der Formel

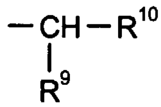

steht,

worin

$R^9$ Wasserstoff oder einen Rest der Formel $CH_2$-OH bedeutet,
$R^{10}$ Phenyl bedeutet, das gegebenenfalls bis zu 2-fach gleich oder verschieden durch Hydroxy, Fluor, Chlor, Brom oder geradkettiges oder verzweigtes Alkyl mit bis zu 3 Kohlenstoffatomen substituiert ist,

und deren Salze.

Besonders bevorzugt sind die erfindungsgemäßen Verbindungen der allgemeinen Formel (I), in welcher

A für Naphthyl, Phenyl, Benzyl, Pyridyl, Imidazolyl, Benzimidazolyl oder Chinolyl steht, die gegebenenfalls - auch über die N-Funktion - bis zu 2-fach gleich oder verschieden durch Fluor, Chlor, Trifluormethyl, Carboxy, Hydroxyl, Nitro, Cyclohexyl, Benzyl, Phenyl, Benzyloxy oder durch geradkettiges oder verzweigtes Alkyl, Alkoxy oder Alkoxycarbonyl mit jeweils bis zu 3 Kohlenstoffatomen substituiert sind, oder für einen Rest der Formel $R^5R^4N$- oder

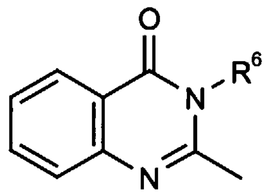 steht,

worin

$R^4$ und $R^5$ gleich oder verschieden sind und Wasserstoff, Phenyl oder Methyl bedeuten,
$R^6$ Wasserstoff, Phenyl oder Methyl bedeutet,

D für ein Sauerstoffatom oder für einen Rest der Formel -CO-, $-(CO)_a-NR^7$, $-(CH_2)_bS-$, $-(CH_2)_c-NR^8$ oder -CH=CH- steht,
worin

a, b und c gleich oder verschieden sind und eine Zahl 0 oder 1 bedeuten,
$R^7$ und $R^8$ gleich oder verschieden sind und Wasserstoff, Methyl, Ethyl, Phenyl, Acetyl oder Benzyl bedeuten, wobei die Ringsysteme gegebenenfalls bis zu 2-fach gleich oder verschieden durch Nitro, Fluor, Chlor, Brom, Trifluormethyl oder durch geradkettiges oder verzweigtes Alkyl oder Alkoxy mit jeweils bis zu 3 Kohlenstoffatomen substituiert sind,

E und L gleich oder verschieden sind und für Wasserstoff, Fluor, Chlor oder Brom stehen,
$R^1$ für Cyclopropyl, Cylcobutyl, Cyclopentyl oder Cyclohexyl steht, oder für geradkettiges oder verzweigtes Alkyl mit bis zu 8 Kohlenstoffatomen steht,
$R^2$ für Wasserstoff oder für geradkettiges oder verzweigtes Alkyl mit bis zu 3 Kohlenstoffatomen steht,
$R^3$ für einen Rest der Formel

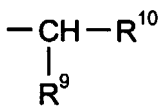 steht,

worin

$R^9$ Wasserstoff oder einen Rest der Formel $CH_2$-OH bedeutet,
$R^{10}$ Phenyl bedeutet, das gegebenenfalls bis zu 2-fach gleich oder verschieden durch Hydroxy, Fluor, Chlor, Methyl oder Ethyl substituiert ist,

und deren Salze.

Außerdem wurde ein Verfahren zur Herstellung der erfindungsgemäßen Verbindungen der allgemeinen Formel (I) gefunden, dadurch gekennzeichnet, dass man
Carbonsäuren der allgemeinen Formel (II)

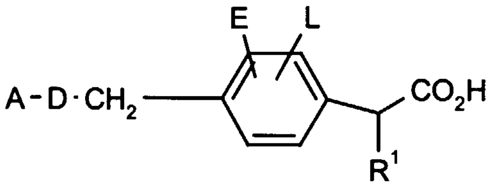 (II),

in welcher

A, D, E, L und $R^1$ die oben angegebene Bedeutung haben,
gegebenenfalls unter vorgeschalteter Aktivierung der Carbonsäurefunktion, mit Phenylglycinolen der allgemeinen Formel (III)

$$HR^2N\text{-}R^3 \quad \text{(III)}$$

in welcher
$R^2$ und $R^3$ die oben angegebene Bedeutung haben,
gegebenenfalls unter Schutzgasatmosphäre, gegebenenfalls in inerten Lösemitteln, in Anwesenheit einer Base und/oder Hilfsmittels umsetzt.

Das erfindungsgemäße Verfahren kann durch folgendes Formelschema beispielhaft erläutert werden:

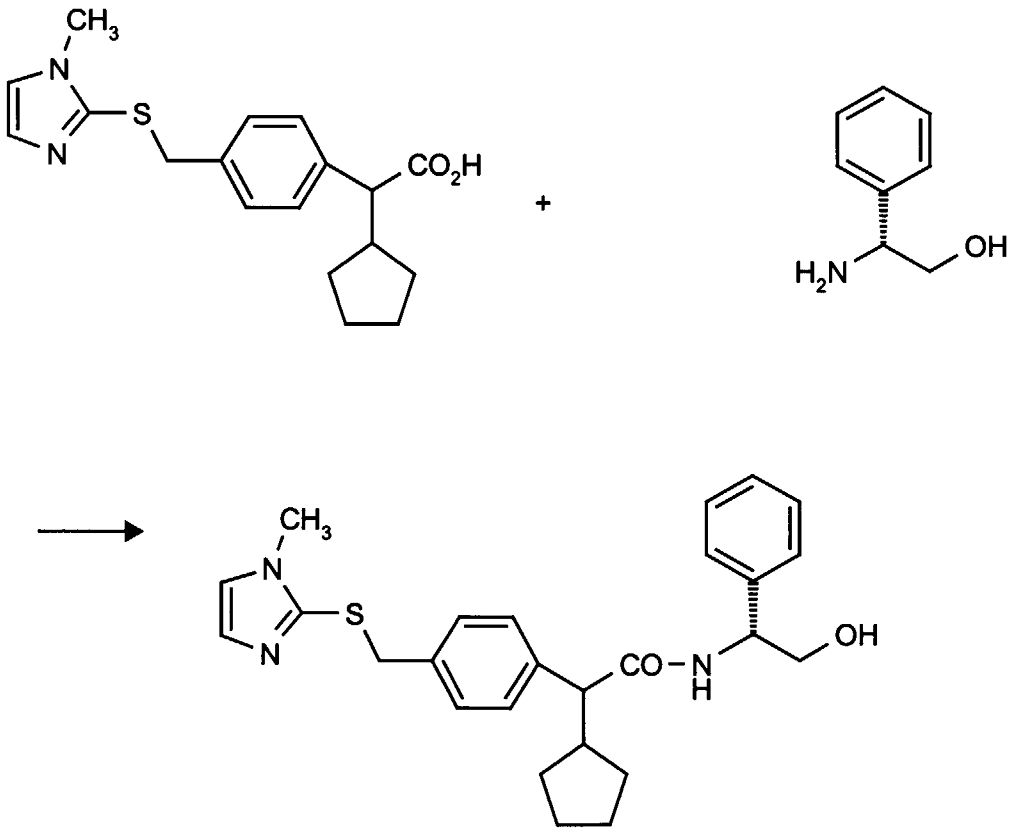

Als Lösemittel eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder

Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton und Dimethylformamid.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliumethanolat, oder Natrium- oder Kaliummethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium. Bevorzugt sind Natrium- und Kaliumcarbonat und Triethylamin.

Die Base wird in einer Menge von 0,6 mol bis 5 mol, bevorzugt von 0,7 mol bis 2 mol, bezogen auf 1 mol der Verbindung der allgemeinen Formel (II) eingesetzt.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Zur Aktivierung der Carbonsäurefunktion eignen sich im allgemeinen Basen und/oder Dehydratisierungsreagenzien wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl- 1,2-oxazolium-3-sulfonat oder Propanphosphonsäureanhydrid oder Isobutylchloroformat oder Benzotriazolyloxy-tris-(dimethylamino)phosphoniumhexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formel (II) sind teilweise bekannt oder neu und können beispielsweise hergestellt werden, indem man

Verbindungen der allgemeinen Formel (IV)

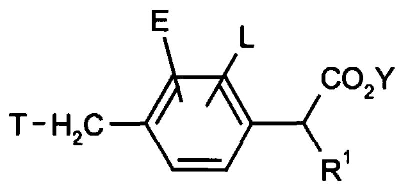

(IV),

in welcher

| | |
|---|---|
| E, L und $R^1$ | die oben angegebene Bedeutung haben, |
| T | für eine typische Abgangsgruppe wie z.B. Chlor, Brom, Jod, Tosylat oder Mesylat, vorzugsweise für Brom steht, und |
| Y | für Wasserstoff, für $(C_1-C_4)$-Alkyl, oder eine andere basenstabile Schutzgruppe steht, |

mit Verbindungen der allgemeinen Formel (V)

$$A\text{-}H \qquad (V)$$

in welcher

A die oben angegebene Bedeutung hat
in inerten Lösemitteln, gegebenenfalls in Anwesenheit einer Base umsetzt,
und anschließend die Ester oder die Schutzgruppe Y nach üblichen Methoden abspaltet.

Die Verbindungen der allgemeinen Formeln (IV) und (V) sind an sich bekannt oder nach üblichen Methoden herstellbar.

Die Verbindungen der allgemeinen Formel (III) sind ebenfalls bekannt oder nach üblichen Methoden herstellbar.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von Koronaren Heizerkrankungen, Herzinsuffizienz, Störungen der Hirnleistung, ischämischen Gehirnerkrankungen, Apoplexie, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muss mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtadsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtadsorption im Vergleich zur Kontrolle (Lösemittelkontrolle ohne Substanz) um 50% inhibiert ist.

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g

zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

**3. Hemmung der intestinalen Triglyceridabsorption in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wässrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus' entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Trigylceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglyceridanstieg} = \frac{\Delta TG_{Substanz} - \Delta TG_{Traganthkontrolle}}{\Delta TG_{Ölbelastung} - \Delta TG_{Traganthkontrolle}} \times 100$$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0,05$) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht (2,5 mg/kg) Triton WR-1339, gelöst in physiologischer Kochsalzlösung, intravenös in die Schwanzvene appliziert. Triton WR- 1339 inhibiert die Lipoproteinlipase und führt somit durch Hemmung des VLDL-Katabolismus zu einem Anstieg des Triglycerid- und Cholsterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Messbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von heteroverknüpften Phenylglycinolamiden der allgemeinen Formel (I) mit einem Glucosidase- und/oder Amylasehemmer zur Behandlung von familiärer Hyperlipidaeamien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

## Abkürzungen im experimentellen Teil

Bn = $CH_2$-$C_6H_5$

Gly =

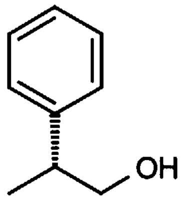

Me = Methyl
Et = Ethyl
cHex = Cyclohexyl
Ph = Phenyl

**Lösemittel**

C/EE = Cyclohexan / Essigester
P/EE = Petrolether / Essigester

**Ausgangsverbindungen**

**Beispiel I**

N-(2-Chlorbenzoyl)-2-cyclopentyl-4-(phenylaminomethyl)-phenyl-essigsäure-tert.butylester

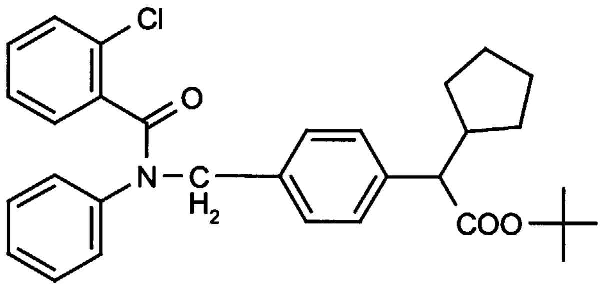

2,3 g (10 mmol) 2-Chlorbenzoesäureanilid werden in 10 ml DMF gelöst, mit 330 mg (11 mmol) NaH (80%ig) deprotoniert (50°C) und anschließend mit 3,5 g (10 mmol) 4-Brommethyl-2-cyclopentyl-essigsäure-tert.butylester (DE 42 00 954 A1) versetzt und über Nacht bei RT gerührt. Es wird eingeengt, in $CH_2Cl_2$ gelöst, mit $H_2O$ gewaschen, eingeengt und der Rückstand an Kieselgel chromatographiert (Cylcohexan / EE = 8:2). Man erhält 3,8 g (75%) als farbloses Harz.

**Beispiel II**

N-(2-Chlorbenzoyl)-2-cyclopentyl-4-(phenylaminomethyl)-phenyl-essigsäure

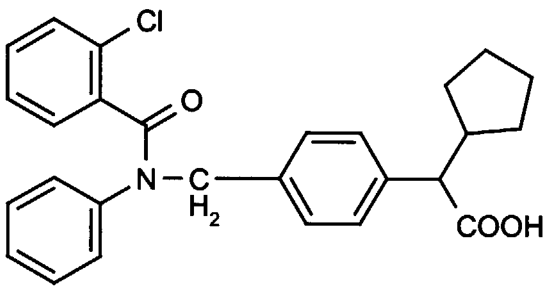

3,5 g (7 mmol) der Verbindung aus Beispiel I werden in 14 ml Dioxan gelöst, mit 2 ml konz. HCl versetzt und 5 h refluxiert. Es wird eingeengt, in $CH_2Cl_2$ aufgenommen und mit Wasser gewaschen. Man erhält 3 g (96%) der Titelverbindung als Öl.

In Analogie zur Vorschrift des Beispiels II werden die in der Tabellen I - III aufgeführten Beispiele hergestellt.

**Tabelle I:**

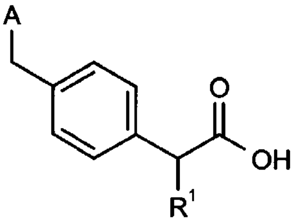

| Bsp.-Nr. | A | $R^1$ | Fp. (°C) | $R_f$ |
|---|---|---|---|---|
| III | 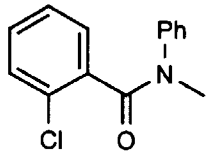 | (R&S) cPent | | 0,28 C/EE 1:1 |
| IV | 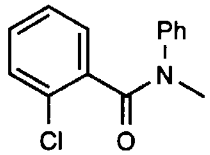 | (R&S) cHept | | 0,3 C/EE 1:1 |
| V | 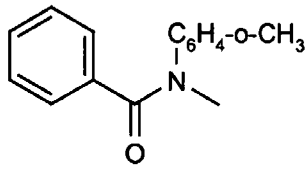 | (R&S) cPent | | 0,32 C/EE 1:1 |
| VI | 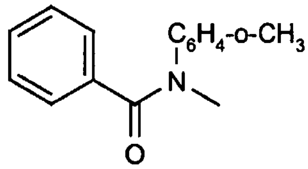 | (R&S) cHept | | 0,46 C/EE 1:1 |
| VII | 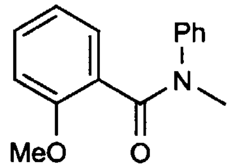 | (R&S) cPent | | 0,23 C/EE 1:1 |
| VIII | 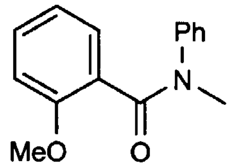 | (R&S) cHept | | 0,19 C/EE 1:1 |

| Bsp.-Nr. | A | $R^1$ | Fp. (°C) | $R_f$ |
|---|---|---|---|---|
| IX | 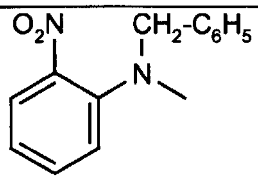 | (R&S) cPent | | 0,22<br>C/EE 1:1 |
| X | 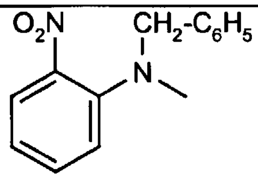 | (R&S) cHept | | 0,28<br>C/EE 1:1 |
| XI | 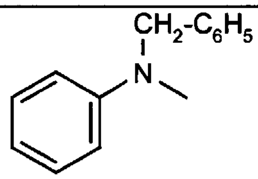 | (R&S) cPent | | 0,25<br>C/EE 1:1 |
| XII | 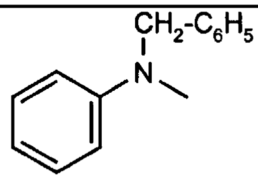 | (R&S) cHept | | 0,33<br>C/EE 1:1 |
| XIII | 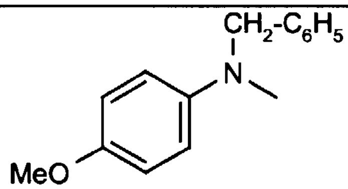 | (R&S) cPent | | 0,29<br>C/EE 1:1 |
| XIV | 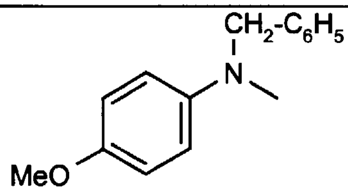 | (R&S) cHept | | 0,35<br>C/EE 1:1 |
| XV | 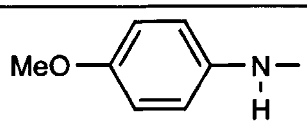 | (R&S) cPent | 151 | |
| XVI | 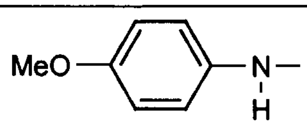 | (R&S) cHept | 155 | |

| Bsp.-Nr. | A | $R^1$ | Fp. (°C) | $R_f$ |
|---|---|---|---|---|
| XVII | 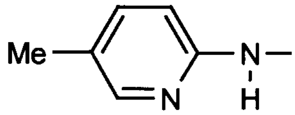 | (R&S) cPent | | 0,05<br>C/EE 1:1 |
| XVIII | 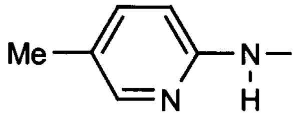 | (R&S) cHept | | 0,05<br>C/EE 1:1 |
| XIX | 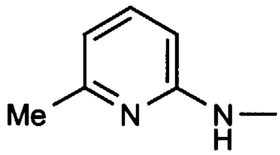 | (R&S) cPent | | 0,11<br>($CH_2Cl_2$/$CH_3OH$ 95:5) |
| XX | 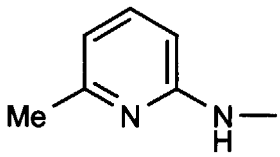 | (R&S) cHept | 211 | |
| XXI | 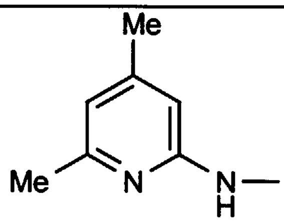 | (R&S) cPent | 211 | |
| XXII | 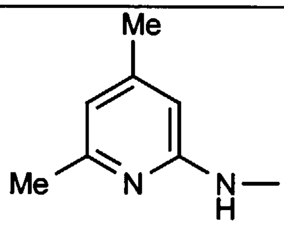 | (R&S) cHept | 190 | |
| XXIII | 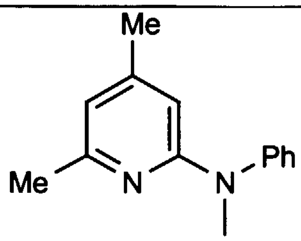 | (R&S) cPent | | 0,58<br>C/EE 1:1 |

| Bsp.-Nr. | A | $R^1$ | Fp. (°C) | $R_f$ |
|---|---|---|---|---|
| XXIV | 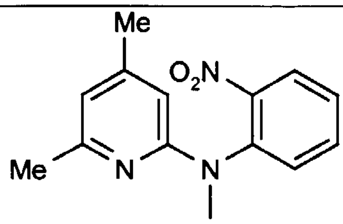 | (R&S) cPent | | 0,11<br>C/EE 7:3 |
| XXV | 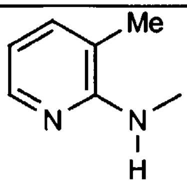 | (R&S) cPent | | 0,13<br>C/EE 1:1 |
| XXVI | 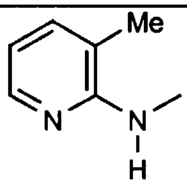 | (R&S) cHept | 244 | |
| XXVII | 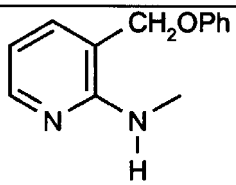 | (R&S) cPent | 159 | |
| XXVIII | 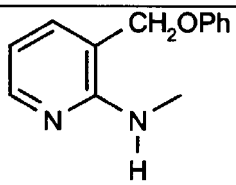 | (R&S) cHept | | 0,06<br>C/EE 7:3 |
| XXIX | 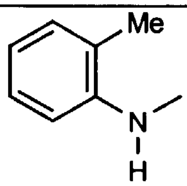 | (R&S) cPent | | |
| XXX | 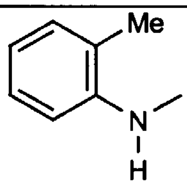 | (R&S) cHept | | 0,15<br>C/EE 7:3 |

| Bsp.-Nr. | A | $R^1$ | Fp. (°C) | $R_f$ |
|---|---|---|---|---|
| XXXI | 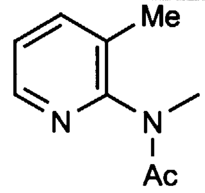 | (R&S) cHept | | |
| XXXII | 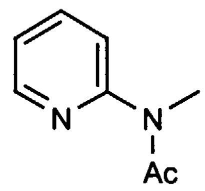 | (R&S) cPent | | |
| XXXIII | 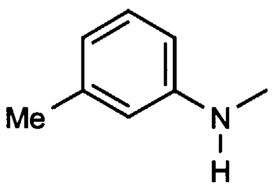 | (R&S) cPent | | 0,42<br>C/EE 1:1 |
| XXXIV | 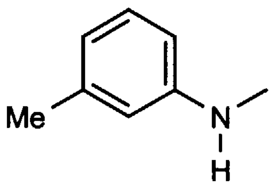 | (R&S) cHept | | 0,15<br>C/EE 7:3 |
| XXXV | 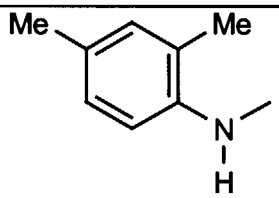 | (R&S) cPent | | 0,13<br>C/EE 7:3 |
| XXXVI | 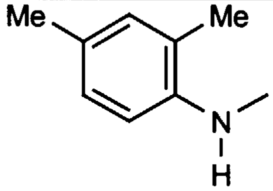 | (R&S) cHept | | 0,18<br>C/EE 7:3 |

**Tabelle II:**

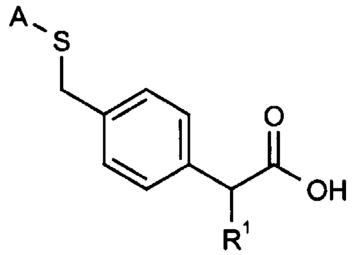

| Bsp.-Nr. | A | $R^1$ | Fp. (°C) | $R_f$ |
|---|---|---|---|---|
| XXXVII | 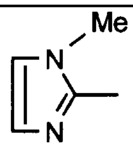 | (R&S) cPent | 158 | |
| XXXVIII | 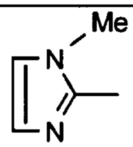 | (R&S) cHept | | 0,1<br>C/EE 3:7 |
| XXXIX | 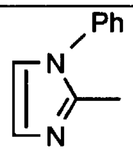 | (R&S) cPent | 184 | |
| XL | 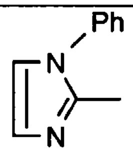 | (R&S) cHept | 139 | |
| XLI | 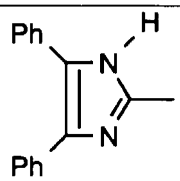 | (R&S) cPent | | 0,27<br>C/EE 1:1 |
| XLII | 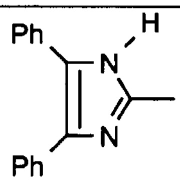 | (R&S) cHept | 172 | |

| Bsp.-Nr. | A | $R^1$ | Fp. (°C) | $R_f$ |
|---|---|---|---|---|
| XLIII | 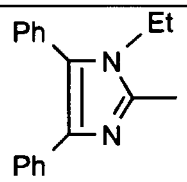 | (R&S) cPent | 205 | |
| XLIV | 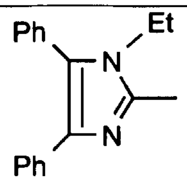 | (R&S) cHept | 190 | |
| XLV | 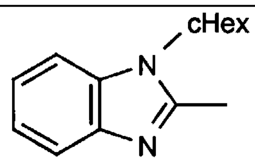 | (R&S) cPent | 197 | |
| XLVI | 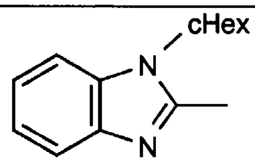 | (R&S) cHept | 213 | |
| XLVII | 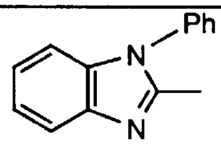 | (R&S) cPent | 204 | |
| XLVIII | 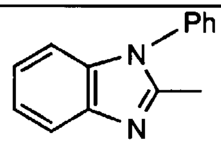 | (R&S) cHept | 205 | |
| XLIX | 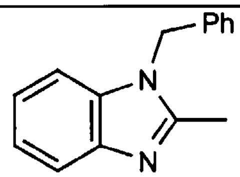 | (R&S) cPent | 173 | |
| L | 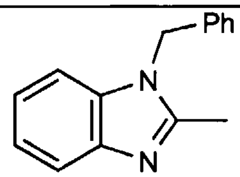 | (R&S) cHept | 146 | |

| Bsp.-Nr. | A | $R^1$ | Fp. (°C) | $R_f$ |
|---|---|---|---|---|
| LI | 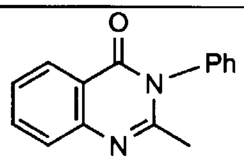 | (R&S) cPent | | 0,3<br>C/EE 7:3 |
| LII | 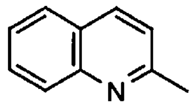 | (R&S) cPent | 119 | |
| LIII | 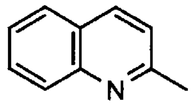 | (R&S) cHept | | 0,21<br>C/EE 7:3 |
| LIV | 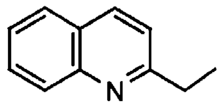 | (R&S) cPent | 136 | |
| LV | 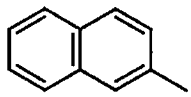 | (R&S) cPent | 129 | |
| LVI | 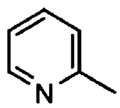 | (R&S) cPent | | 0,42<br>C/EE 1:1 |
| LVII | 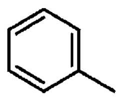 | (R&S) cPent | 132 | |
| LVIII | 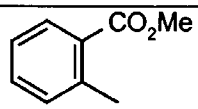 | (R&S) cPent | 183 | |
| LIX | 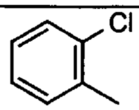 | (R&S) cPent | 134 | |
| LX | 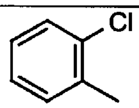 | (R&S) cHept | | 0,30<br>C/EE 7:3 |
| LXI | 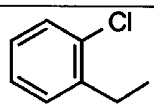 | (R&S) cPent | | 0,5<br>C/EE 1:1 |

| Bsp.-Nr. | A | $R^1$ | Fp. (°C) | $R_f$ |
|---|---|---|---|---|
| LXII | 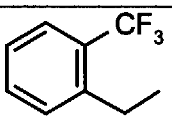 | (R&S) cPent | | 0,39<br>C/EE 1:1 |
| LXIII | 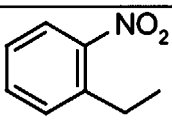 | (R&S) cPent | | 0,29<br>C/EE 1:1 |

**Tabelle III:**

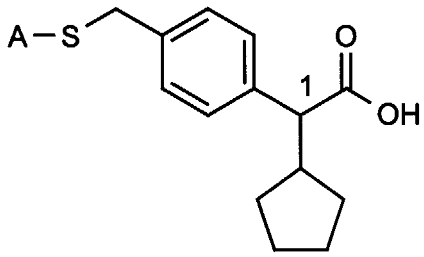

| **Bsp.-Nr.** | **A** | **1** | **Fp. (°C)** | **$R_f$** |
|---|---|---|---|---|
| LXIV | 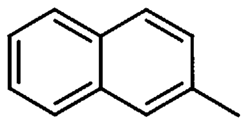 | R&S | | |
| LXV | 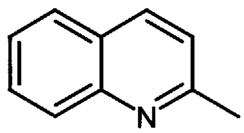 | R&S | | |
| LXVI | 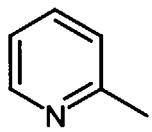 | R&S | | |
| LXVII | 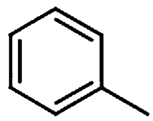 | R&S | | |
| LXVIII | 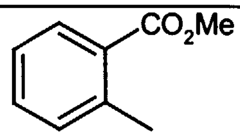 | R&S | | |

__Herstellungsbeispiele__

**Beispiel 1**

N-(2-Chlorbenzoyl)-2-cyclopentyl-4-(phenylamino-methyl)-phenyl-[(N'-(2-hydroxy)-1-(R)-phenylethyl)]-2-essigsäureamid

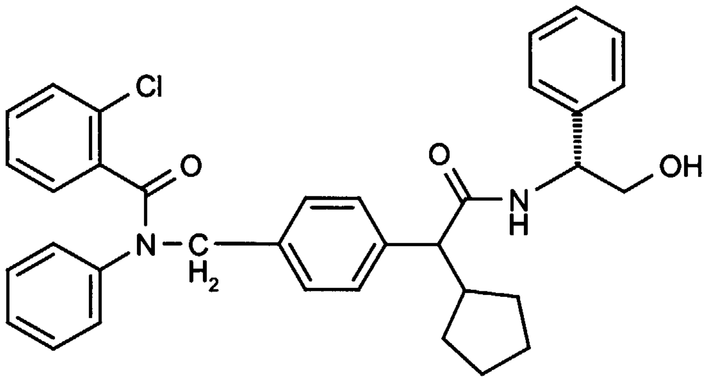

1,34 g (3 mmol) der Verbindung aus Beispiel II werden mit 0,412 g (3 mmol) R-(-)-2-Phenylglycinol (Aldrich) in 30 ml $CH_2Cl_2$ gelöst, anschließend werden 0,446 mg (3,3 mmol) 1-Hydroxy-1H-benzotriazol Hydrat (Aldrich) zugegeben. Nach Zugabe von 662 mg (3,45 mmol) N'-(3-Dimethylaminopropyl)-N-ethyl-carbodiimid-hydrochlorid (Aldrich) und 0,8 ml Triethylamin wird über Nacht bei RT gerührt. Es wird mit $CH_2Cl_2$ verdünnt, je einmal mit $NH_4Cl$-Lösung und $NaHCO_3$-Lösung gewaschen, getrocknet und einrotiert. Es wird mit Cyclohexan / Essigester (1:1) chromatographiert.
Ausbeute: 1,67 g (98%).
$R_f$ = 0,17 (Cyclohexan / Essigester = 1:1)

In Analogie zur Vorschrift des Beispiels 1 werden die in den Tabellen 1 - 3 aufgeführten Verbindungen über die entsprechenden Vorstufen (analog der Beispiele I und II) hergestellt:

**Tabelle 1:**

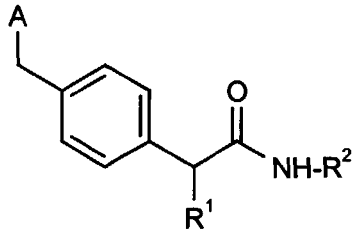

| Bsp.-Nr. | A | $R^1$ | $R^2$ | Fp. (°C) | $R_f$ |
|---|---|---|---|---|---|
| 2 | 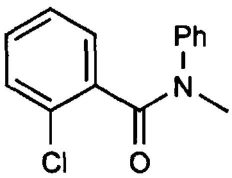 | (R&S) cPent | Bn | 176-177 | |
| 3 | 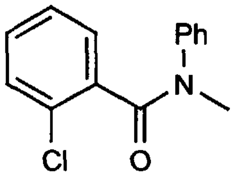 | (R&S) cHept | R-Gly | | 0,23 C/EE 1:1 |
| 4 | 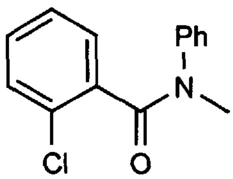 | (R&S) cHept | Bn | 171-172 | |
| 5 | 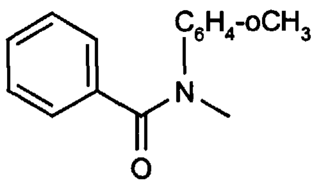 | (R&S) cPent | R-Gly | | 0,21 C/EE 1:1 |
| 6 | 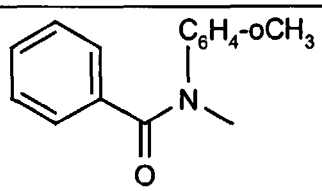 | (R&S) cPent | Bn | 182-183 | |

| Bsp.-Nr. | A | $R^1$ | $R^2$ | Fp. (°C) | $R_f$ |
|---|---|---|---|---|---|
| 7 | 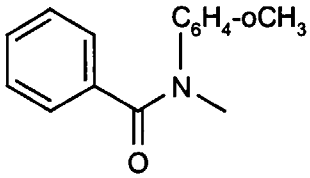 | (R&S) cHept | R-Gly | | 0,26 C/EE 1:1 |
| 8 | 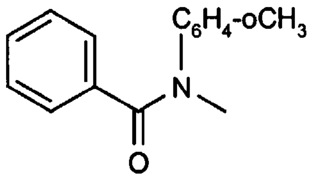 | (R&S) cHept | Bn | 146-147 | |
| 9 | 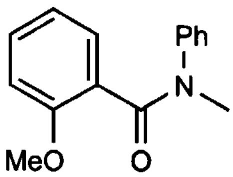 | (R&S) cPent | R-Gly | | 0,13 C/EE 1:1 |
| 10 | 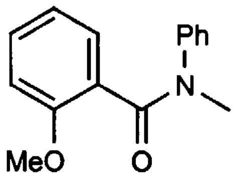 | (R&S) cPent | Bn | 171 | |
| 11 | 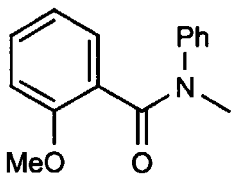 | (R&S) cHept | R-Gly | | 0,13 C/EE 1:1 |
| 12 | 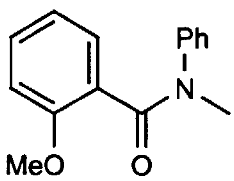 | (R&S) cHept | Bn | 144 | |
| 13 | 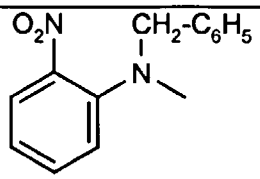 | (R&S) cPent | R-Gly | | 0,14 C/EE 1:1 |

| Bsp.-Nr. | A | $R^1$ | $R^2$ | Fp. (°C) | $R_f$ |
|---|---|---|---|---|---|
| 14 | 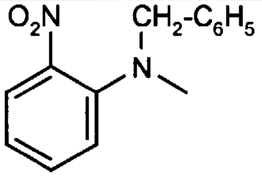 | (R&S) cPent | Bn | | 0,39 C/EE 1:1 |
| 15 | 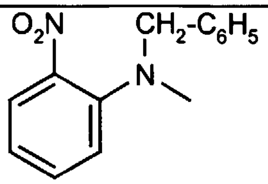 | (R&S) cHept | R-Gly | | 0,17 C/EE 1:1 |
| 16 | 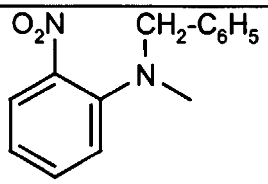 | (R&S) cHept | Bn | | 0,44 C/EE 1:1 |
| 17 | 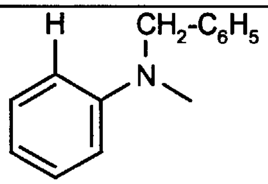 | (R&S) cPent | R-Gly | | 0,18 C/EE 1:1 |
| 18 | 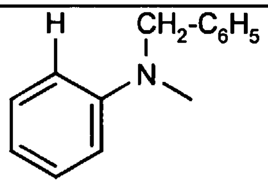 | (R&S) cPent | Bn | 160 | |
| 19 | 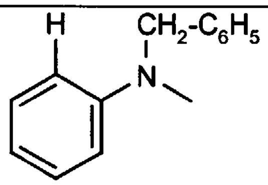 | (R&S) cHept | R-Gly | | 0,27 C/EE 1:1 |
| 20 | 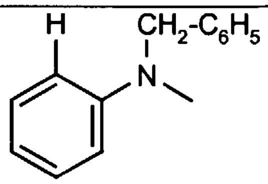 | (R&S) cHept | Bn | 166 | |

| Bsp.-Nr. | A | $R^1$ | $R^2$ | Fp. (°C) | $R_f$ |
|---|---|---|---|---|---|
| 21 | 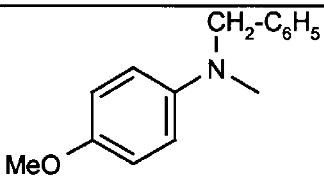 | (R&S) cPent | R-Gly | | 0,16 C/EE 1:1 |
| 22 | 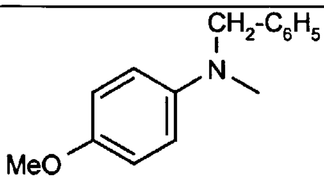 | (R&S) cPent | Bn | 159 | |
| 23 | 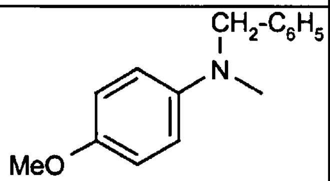 | (R&S) cHept | R-Gly | | 0,22 C/EE 1:1 |
| 24 | 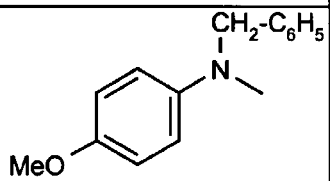 | (R&S) cHept | Bn | 171 | |
| 25 | 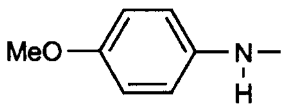 | (R&S) cPent | R-Gly | | 0,21 C/EE 1:1 |
| 26 | 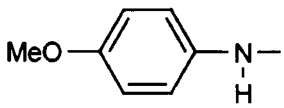 | (R&S) cPent | Bn | 146-147 | |
| 27 | 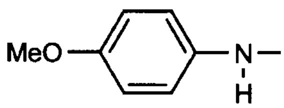 | (R&S) cHept | R-Gly | | 0,32 C/EE 1:1 |
| 28 | 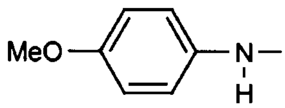 | (R&S) cHept | Bn | 105 | |

| Bsp.-Nr. | A | $R^1$ | $R^2$ | Fp. (°C) | $R_f$ |
|---|---|---|---|---|---|
| 29 | 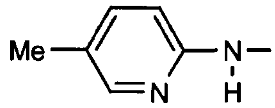 | (R&S) cPent | R-Gly | | 0,31 C/EE 4:6 |
| 30 | 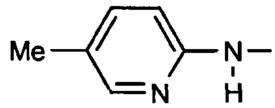 | (R&S) cPent | Bn | 133 | |
| 31 | 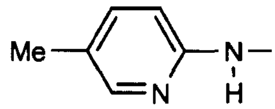 | (R&S) cHept | R-Gly | | 0,1 C/EE 1:1 |
| 32 | 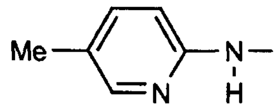 | (R&S) cHept | Bn | 110-111 | |
| 33 | 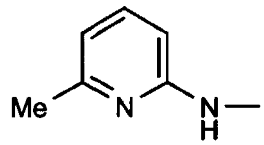 | (R&S) cPent | R-Gly | | 0,39 C/EE 4:6 |
| 34 | 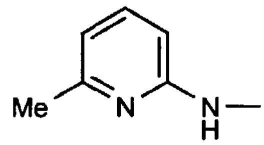 | (R&S) cPent | Bn | 159-160 | |
| 35 | 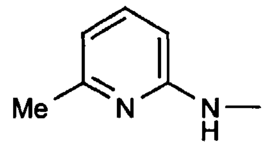 | (R&S) cHept | R-Gly | | 0,17 C/EE 4:6 |
| 36 | 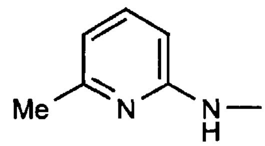 | (R&S) cHept | Bn | 151-152 | |
| 37 | 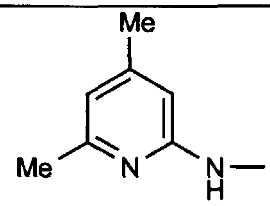 | (R&S) cPent | R-Gly | | 0,08 C/EE 1:1 |

| Bsp.-Nr. | A | $R^1$ | $R^2$ | Fp. (°C) | $R_f$ |
|---|---|---|---|---|---|
| 38 | 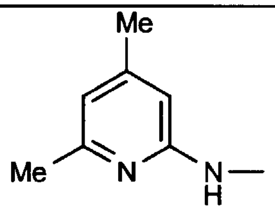 | (R&S) cPent | Bn | 161-162 | |
| 39 | 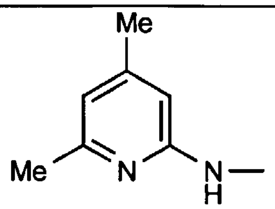 | (R&S) cHept | R-Gly | 129-130 | |
| 40 | 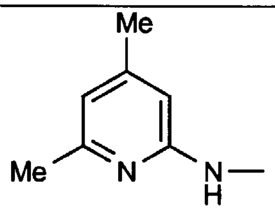 | (R&S) cHept | Bn | 155-156 | |
| 41 | 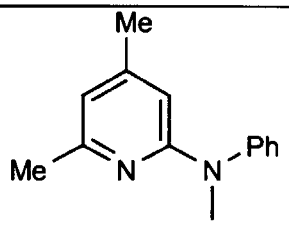 | (dia A) cPent | R-Gly | 177 | |
| 42 | 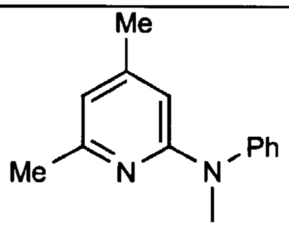 | (dia B) cPent | Bn | | 0,40 C/EE 1:1 |
| 43 | 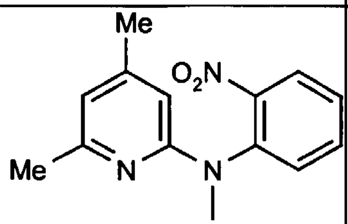 | (R&S) cPent | R-Gly | | 0,23 C/EE 7:3 |

| Bsp.-Nr. | A | $R^1$ | $R^2$ | Fp. (°C) | $R_f$ |
|---|---|---|---|---|---|
| 44 | 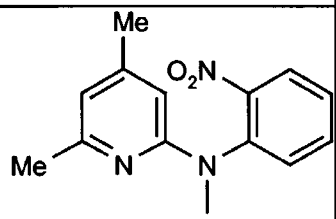 | (R&S) cPent | Bn | | 0,20 C/EE 7:3 |
| 45 | 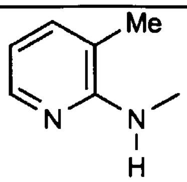 | (R&S) cPent | R-Gly | | 0,15 C/EE 1:1 |
| 46 | 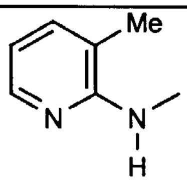 | (R&S) cPent | Bn | | 0,23 C/EE 7:3 |
| 47 | 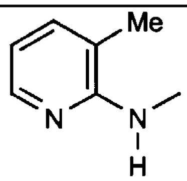 | (R&S) cHept | R-Gly | | 0,14 C/EE 1:1 |
| 48 | 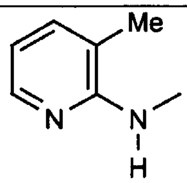 | (R&S) cHept | Bn | | 0,23 C/EE 7:3 |
| 49 | 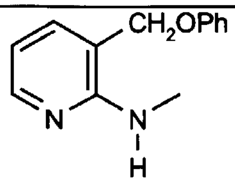 | (R&S) cPent | R-Gly | 134-135 | |
| 50 | 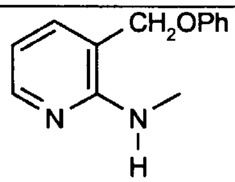 | (R&S) cPent | Bn | | 0,23 C/EE 7:3 |

| Bsp.-Nr. | A | $R^1$ | $R^2$ | Fp. (°C) | $R_f$ |
|---|---|---|---|---|---|
| 51 | 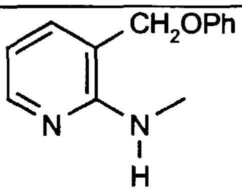 | (R&S) cHept | R-Gly | | 0,25 C/EE 1:1 |
| 52 | 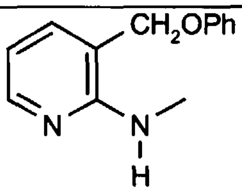 | (R&S) cHept | Bn | | 0,31 C/EE 7:3 |
| 53 | 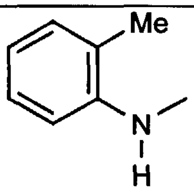 | (R&S) cPent | R-Gly | | 0,37 C/EE 1:1 |
| 54 | 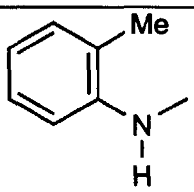 | (R&S) cPent | Bn | | 0,45 C/EE 7:3 |
| 55 | 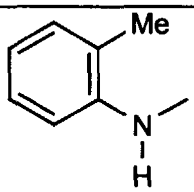 | (R&S) cHept | R-Gly | | 0,07 C/EE 7:3 |
| 56 | 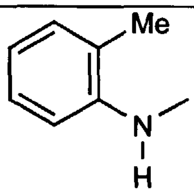 | (R&S) cHept | Bn | 149-150 | |
| 57 | 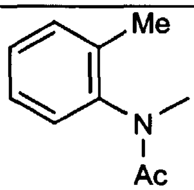 | (R&S) cHept | Bn | | 0,08 C/EE 4:6 |

| Bsp.-Nr. | A | $R^1$ | $R^2$ | Fp. (°C) | $R_f$ |
|---|---|---|---|---|---|
| 58 | 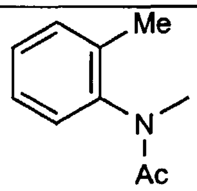 | (R&S) cPent | R-Gly | | 0,05 C/EE 4:6 |
| 59 | 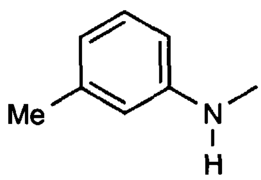 | (R&S) cPent | R-Gly | | 0,34 C/EE 1:1 |
| 60 | 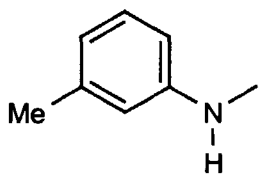 | (R&S) cPent | Bn | | 0,39 C/EE 7:3 |
| 61 | 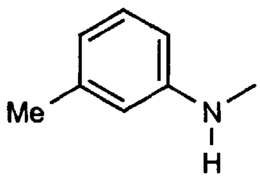 | (R&S) cHept | R-Gly | | 0,13 C/EE 7:3 |
| 62 | 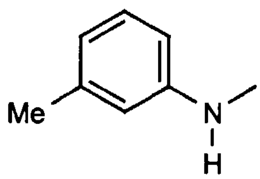 | (R&S) cHept | Bn | | 0,33 C/EE 7:3 |
| 63 | 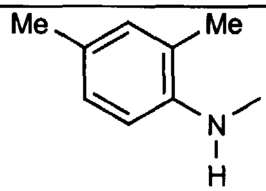 | (R&S) cPent | R-Gly | | 0,37 C/EE 1:1 |
| 64 | 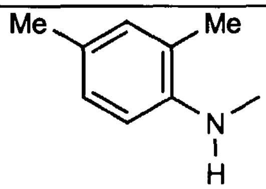 | (R&S) cPent | Bn | | 0,25 C/EE 7:3 |

| Bsp.-Nr. | A | $R^1$ | $R^2$ | Fp. (°C) | $R_f$ |
|---|---|---|---|---|---|
| 65 | 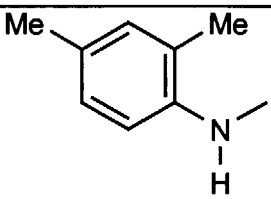 | (R&S) cHept | R-Gly | | 0,45 C//EE 1:1 |
| 66 | 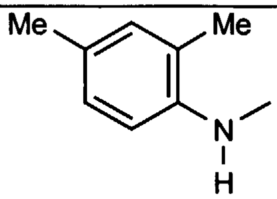 | (R&S) cHept | Bn | | 0,3 C/EE 7:3 |

C/EE = Cyclohexan : Essigester

**Tabelle 2:**

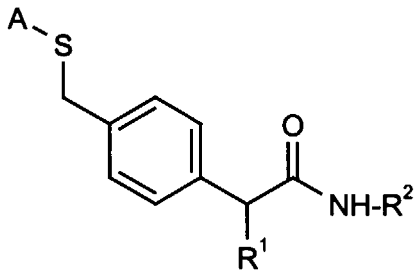

| **Bsp.-Nr.** | **A** | **$R^1$** | **$R^2$** | **Fp. (°C)** | **$R_f$** |
|---|---|---|---|---|---|
| 67 | 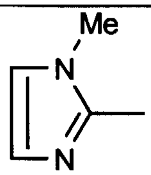 | (R&S) cPent | R-Gly | | 0,05 C/EE 3:7 |
| 68 | 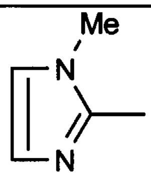 | (R&S) cPent | Bn | | 0,08 C/EE 1:1 |
| 69 | 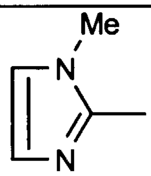 | (R&S) cHept | R-Gly | 155-156 | |
| 70 | 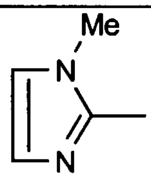 | (R&S) cHept | Bn | | 0,09 C/EE 1:1 |
| 71 | 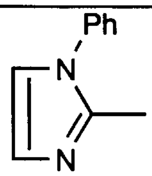 | (R&S) cPent | R-Gly | | 0,09 C/EE 1:1 |

| Bsp.-Nr. | A | $R^1$ | $R^2$ | Fp. (°C) | $R_f$ |
|---|---|---|---|---|---|
| 72 | 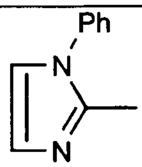 | (R&S) cPent | Bn | 108-109 | |
| 73 | 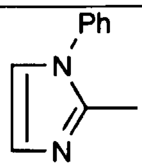 | (R&S) cHept | R-Gly | | 0,12 C/EE 1:1 |
| 74 | 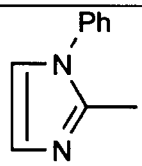 | (R&S) cHept | Bn | 144-145 | |
| 75 | 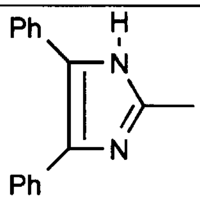 | (R&S) cPent | R-Gly | | 0,22 C/EE 1:1 |
| 76 | 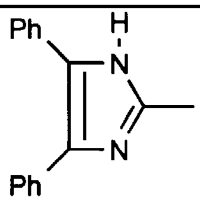 | (R&S) cPent | Bn | 140-141 | |
| 77 | 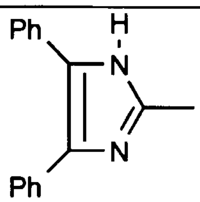 | (R&S) cHept | R-Gly | | 0,28 C/EE 1:1 |
| 78 | 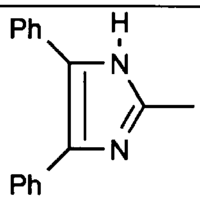 | (R&S) cHept | Bn | 186-187 | |

| Bsp.-Nr. | A | $R^1$ | $R^2$ | Fp. (°C) | $R_f$ |
|---|---|---|---|---|---|
| 79 | 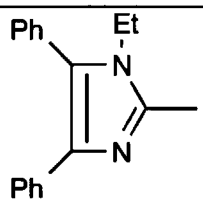 | (R&S) cPent | R-Gly | | 0,25 C/EE 1:1 |
| 80 | 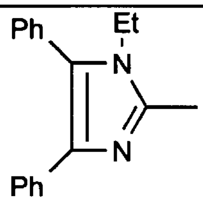 | (R&S) cPent | Bn | 135-136 | |
| 81 | 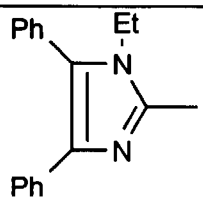 | (R&S) cHept | R-Gly | | 0,33 C/EE 1:1 |
| 82 | 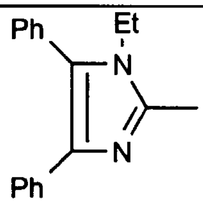 | (R&S) cHept | Bn | 173-174 | |
| 83 | 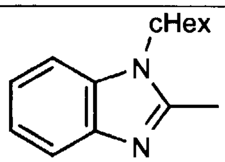 | (R&S) cPent | R-Gly | | 0,26 C/EE 1:1 |
| 84 | 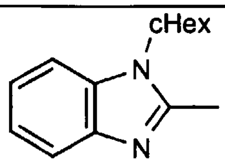 | (R&S) cPent | Bn | | 0,28 C/EE 7:3 |
| 85 | 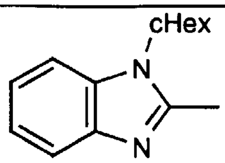 | (R&S) cHept | R-Gly | | 0,37 C/EE 1:1 |

| Bsp.-Nr. | A | $R^1$ | $R^2$ | Fp. (°C) | $R_f$ |
|---|---|---|---|---|---|
| 86 | 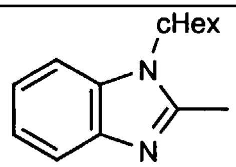 | (R&S) cHept | Bn | | 0,39 C/EE 7:3 |
| 87 | 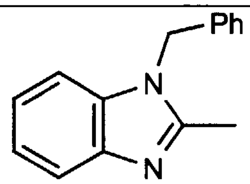 | (R&S) cPent | R-Gly | | 0,29 C/EE 1:1 |
| 88 | 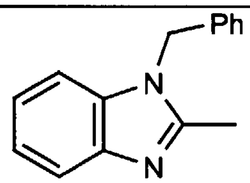 | (R&S) cPent | Bn | 138-139 | |
| 89 | 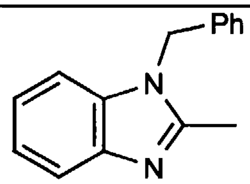 | (R&S) cHept | R-Gly | | 0,35 C/EE 1:1 |
| 90 | 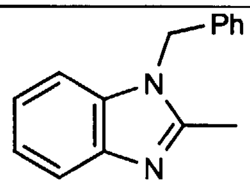 | (R&S) cHept | Bn | 164-165 | |
| 91 | 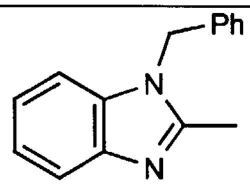 | (R&S) cPent | R-Gly | 181-182 | |
| 92 | 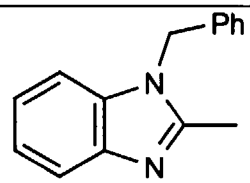 | (R&S) cPent | Bn | 154-155 | |

| Bsp.-Nr. | A | $R^1$ | $R^2$ | Fp. (°C) | $R_f$ |
|---|---|---|---|---|---|
| 93 | 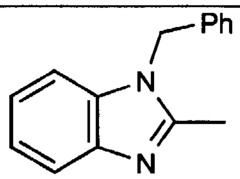 | (R&S) cHept | R-Gly | | 0,32 C/EE 1:1 |
| 94 | 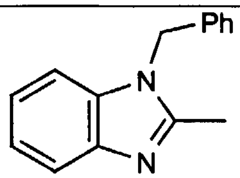 | (R&S) cHept | Bn | 158-159 | |
| 95 | 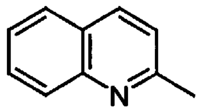 | (R&S) cPent | R-Gly | | 0,25 C/EE 1:1 |
| 96 | 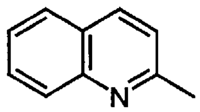 | (dia A) cPent | R-Gly | 188 | |
| 97 | 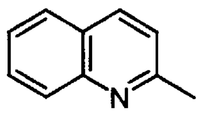 | (dia B) cPent | R-Gly | 143 | |
| 98 | 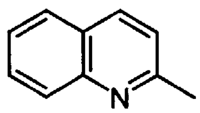 | (R&S) cHept | R-Gly | 139-140 | |
| 99 | 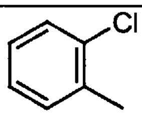 | (R&S) cPent | Bn | | 0,43 C/EE 7:3 |
| 100 | 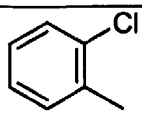 | (R&S) cHept | Bn | | 0,46 C/EE 7:3 |
| 101 | 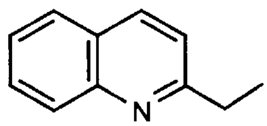 | (R&S) cPent | R-Gly | | 0,33 C/EE 1:1 |

| Bsp.-Nr. | A | $R^1$ | $R^2$ | Fp. (°C) | $R_f$ |
|---|---|---|---|---|---|
| 102 | 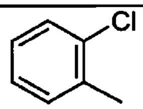 | (R&S) cPent | R-Gly | 150-151 | |
| 103 | 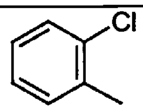 | (R&S) cPent | R-Gly | 155-156 | |
| 104 | 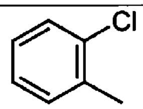 | (R&S) cPent | R-Gly | 167-168 | |
| 105 | 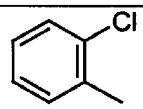 | (R&S) cPent | R-Gly | 174-175 | |
| 106 | 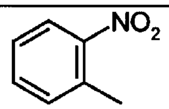 | (R&S) cPent | R-Gly | 158-160 | |
| 107 | 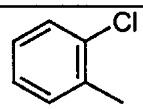 | (R&S) cHept | R-Gly | 156-157 | |
| 108 | 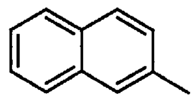 | (dia A) cPent | R-Gly | 164 | |
| 109 | 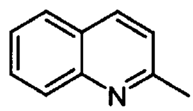 | (dia B) cPent | R-Gly | | 0,32 C/EE 1:1 |
| 110 | 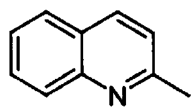 | (dia A) cPent | R-Gly | 178 | |
| 111 | 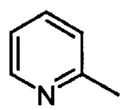 | (dia B) cPent | R-Gly | | 0,32 C/EE 1:1 |
| 112 | 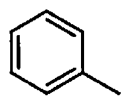 | (R&S) cPent | R-Gly | 150 | |

**Tabelle 3:**

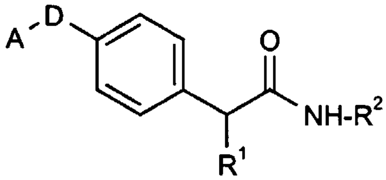

| Bsp.-Nr. | A | D | $R^1$ | $R^2$ | Fp. (°C) | $R_f$ |
|---|---|---|---|---|---|---|
| 113 | 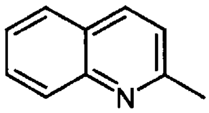 | O | (R&S) cPent | R-Gly | | 0,32<br>$CH_2Cl_2/CH_3OH$<br>100:10 |
| 114 | 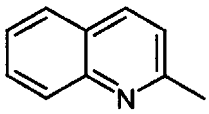 | $CH_2$-S | (R&S) cPent | R-Gly | 159 | |
| 115 | 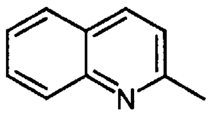 | $CH_2$-NH | (R&S) cPent | R-Gly | | 0,10<br>C/EE 1:1 |
| 116 | 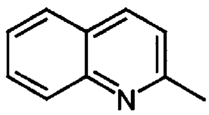 | $CH_2$-NH | (R&S) cPent | Bn | | 0,35<br>C/EE 1:1 |
| 117 | 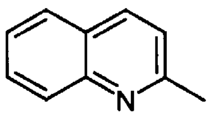 | CO-NH | (R&S) cPent | R-Gly | | 0,22<br>C/EE 1:1 |
| 118 | 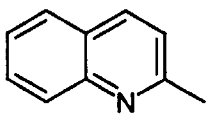 | CO-NH | (R&S) cPent | Bn | 181 | |
| 119 | 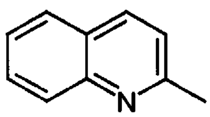 | O-$CH_2$ | (R&S) cPent | R-Gly | 155 | |
| 120 | 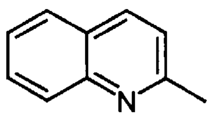 | CH=CH | (R&S) cPent | R-Gly | | 0,6<br>$CH_2Cl_2$/EE 1:1 |

| Bsp.-Nr. | A | D | $R^1$ | $R^2$ | Fp. (°C) | $R_f$ |
|---|---|---|---|---|---|---|
| 121 | $Me_2NCOS$- | - | (R&S) cPent | R-Gly | | 0,10 C/EE 1:1 |
| 122 | 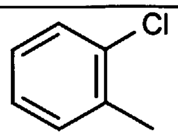 | $CH_2$-S | (dia A) cPent | R-Gly | 145 | |
| 123 | 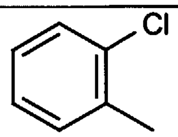 | $CH_2$-S | (dia B) cPent | R-Gly | 144 | |
| 124 | 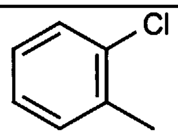 | $CH_2$-NH | (R&S) cPent | R-Gly | | 0,25 C/EE 1:1 |
| 125 | 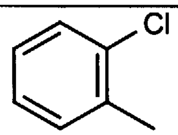 | $CH_2$-NH | (R&S) cPent | Bn | 112 | |
| 126 | 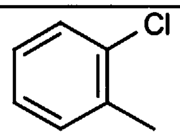 | $CH_2$-N(Ac) | (R&S) cPent | R-Gly | 147 | |
| 127 | 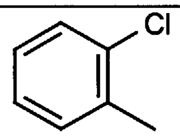 | $CH_2$-N(Ac) | (R&S) cPent | Bn | 103 | |
| 128 | 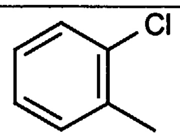 | CO-NH | (R&S) cPent | | 235 | |
| 129 | 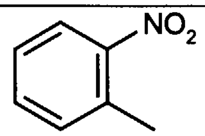 | CO-NH | (R&S) cPent | R-Gly | 243 | |

**Tabelle 4:**

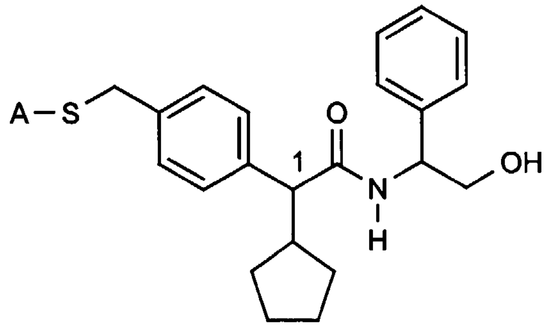

| Bsp.-Nr. | A | 1 | Fp. (°C) | $R_f$ |
|---|---|---|---|---|
| 130 | 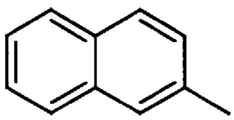 | R&S | | 0,14 C/EE 4:6 |
| 131 | 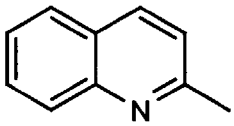 | dia A | 137-139 | |
| 132 | 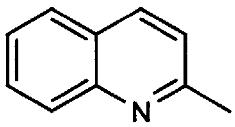 | dia B | | 0,08 C/EE 1:1 |
| 133 | 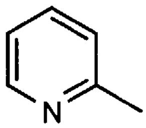 | R&S | | 0,06 C/EE 4:6 |
| 134 | 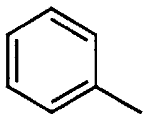 | R&S | 166-167 | |
| 135 | 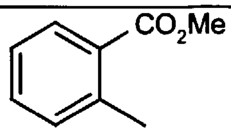 | R&S | | 0,09 C/EE 4:6 |

**Patentansprüche**

**1.** Verbindungen der Formel

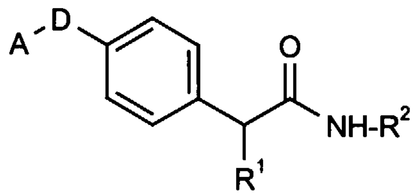

| Bsp.-Nr. | A | D | $R^1$ | $R^2$ |
|---|---|---|---|---|
| a | 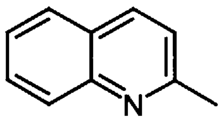 | O | (R&S) cPent | R-Gly |
| b | 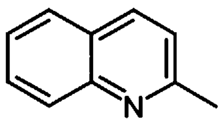 | $CH_2$-S | (R&S) cPent | R-Gly |
| c | 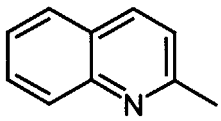 | $CH_2$-NH | (R&S) cPent | R-Gly |
| d | 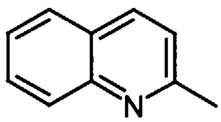 | $CH_2$-NH | (R&S) cPent | Bn |
| e | 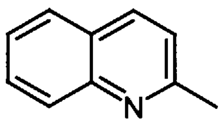 | CO-NH | (R&S) cPent | R-Gly |
| f | 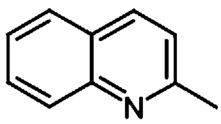 | CO-NH | (R&S) cPent | Bn |
| g | 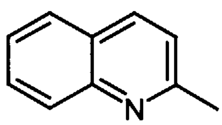 | CH=CH | (R&S) cPent | R-Gly |
| h | $Me_2NCOS$- | - | (R&S) cPent | R-Gly |

| Bsp.-Nr. | A | D | $R^1$ | $R^2$ |
|---|---|---|---|---|
| i | 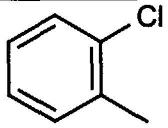 | $CH_2$-S | (dia A) cPent | R-Gly |
| j | 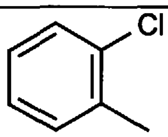 | $CH_2$-S | (dia B) cPent | R-Gly |
| k | 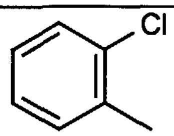 | $CH_2$-NH | (R&S) cPent | R-Gly |
| l | 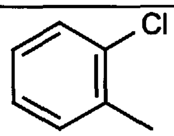 | $CH_2$-NH | (R&S) cPent | Bn |
| m | 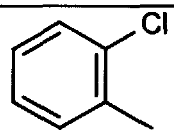 | $CH_2$-N(Ac) | (R&S) cPent | R-Gly |
| n | 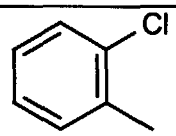 | $CH_2$-N(Ac) | (R&S) cPent | Bn |
| o | 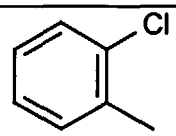 | CO-NH | (R&S) cPent | |
| p | 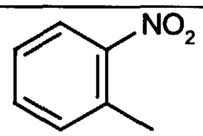 | CO-NH | (R&S) cPent | R-Gly |

2. Arzneimittel enthaltend mindestens eine Verbindung gemäß Anspruch 1 und ein pharmakologisch unbedenkliches Formulierungsmittel.

3. Arzneimittel gemäß Anspruch 2 zur Behandlung von Atherosklerose.

4. Arzneimittel gemäß Anspruch 2 zur Behandlung von Adipositas.

5. Verwendung von Verbindungen gemäß Anspruch 1 zur Herstellung von Arzneimitteln.

6. Verwendung gemäß Anspruch 5 zur Herstellung von Arzneimitteln zur Behandlung von Atherosklerose.

**7.** Verwendung gemäß Anspruch 5 zur Herstellung von Arzneimitteln zur Behandlung von Adipositas.

**8.** Verwendung von Verbindungen gemäß Anspruch 1 zur Herstellung von Arzneimitteln zur Verminderung oder vollständigen Inhibierung der Bildung und/oder der Freisetzung von ApoB-100-assoziierten Lipoproteinen.

Europäisches Patentamt

# EUROPÄISCHER RECHERCHENBERICHT

Nummer der Anmeldung
EP 00 10 9077

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | EP 0 344 519 A (BAYER AG)<br>6. Dezember 1989 (1989-12-06)<br>* Seite 16, Zeile 12; Ansprüche; Beispiel 34 *<br>----- | 1-8 | C07D213/74<br>C07D213/70<br>C07D213/75<br>C07C233/22<br>C07D233/84<br>C07D215/36<br>C07D235/28<br>C07D239/95<br>A61K31/44<br>A61K31/41<br>A61K31/47<br>A61K31/165 |

RECHERCHIERTE SACHGEBIETE (Int.Cl.7)
C07D
C07C
A61K

Der vorliegende Recherchenbericht wurde für alle Patentansprüche erstellt

| Recherchenort | Abschlußdatum der Recherche | Prüfer |
|---|---|---|
| DEN HAAG | 16. Juni 2000 | Bosma, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 00 10 9077

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-06-2000

| Im Recherchenbericht angeführtes Patentdokument | Datum der Veröffentlichung | Mitglied(er) der Patentfamilie | Datum der Veröffentlichung |
|---|---|---|---|
| EP 0344519 A | 06-12-1989 | DE 3900261 A | 07-12-1989 |
| | | AT 88183 T | 15-04-1993 |
| | | AU 616269 B | 24-10-1991 |
| | | AU 3527089 A | 07-12-1989 |
| | | CA 1333802 A | 03-01-1995 |
| | | CN 1038641 A,B | 10-01-1990 |
| | | DE 58904042 D | 19-05-1993 |
| | | DK 263889 A | 01-12-1989 |
| | | ES 2053864 T | 01-08-1994 |
| | | FI 892603 A,B, | 01-12-1989 |
| | | HK 38895 A | 24-03-1995 |
| | | HU 50780 A,B | 28-03-1990 |
| | | HU 9500147 A | 28-07-1995 |
| | | IE 61922 B | 30-11-1994 |
| | | IL 90435 A | 21-02-1993 |
| | | JP 2019359 A | 23-01-1990 |
| | | JP 2693576 B | 24-12-1997 |
| | | JP 10053577 A | 24-02-1998 |
| | | KR 131202 B | 17-04-1998 |
| | | NO 174889 B | 18-04-1994 |
| | | NZ 229310 A | 25-10-1991 |
| | | PT 90675 A,B | 30-11-1989 |
| | | SG 12795 G | 16-06-1995 |
| | | US 4970215 A | 13-11-1990 |
| | | ZA 8904093 A | 27-02-1991 |

EPO FORM P0461

Für nähere Einzelheiten zu diesem Anhang : siehe Amtsblatt des Europäischen Patentamts, Nr.12/82